# FASCICULE DE BREVET EUROPEEN

(11) **EP 1 450 766 B1**
(45) Date de publication et mention de la délivrance du brevet: **22.04.2015**
(21) Numéro de dépôt: 02793162.5
(22) Date de dépôt: 10.10.2002
(51) Int. Cl.: A61K 9/16

(54) **MICROSPHERES BIODEGRADABLES A LIBERATION PROLONGEE ET LEUR PROCEDE DE PREPARATION**
BIOLOGISCH ABBAUBARE MIKROKÜGELCHEN MIT VERLÄNGERTER FREISETZUNG UND VERFAHREN ZU IHRER HERSTELLUNG
PROLONGED RELEASE BIODEGRADABLE MICROSPHERES AND METHOD FOR PREPARING SAME

(30) Priorité: 10.10.2001 FR 0113031; 10.10.2001 ES 200102261
(43) Date de publication de la demande: 01.09.2004
(73) Titulaire: PIERRE FABRE MEDICAMENT, 92100 Boulogne-Billancourt (FR)
(72) Inventeur: FERRET, Eulalia, E-08820 El Prat de Llobregat (ES); ASIN, Miguel Angel, E-08290 Cerdanyola del Valles (ES); GARCIA, Jésus, E-08015 Barcelona (ES); TARIN, Pere, E-08201 Sabadell (ES); AROLA, Rosa, E-08015 Barcelone (ES); RUTLLAN, Montserrat, E-8036 Barcelona (ES); PEREZ, Amadeo, E-08028 Barcelone (ES)
(74) Mandataire: Regimbeau
(86) Numéro de dépôt international: PCT/FR2002/003447
(87) Numéro de publication internationale: WO 2003/030870

(56) Documents cités:
- EP-A- 0 469 520
- EP-A- 0 582 459
- WO-A-95/28149
- T. UCHIDA ET AL.: "Optimization of Preparative Cnditions for Polylactide (PLA) Microspheres Containing Ovalbumin" CHEMICAL AND PHARMACEUTICAL BULLETIN, vol. 43, no. 9, 1 septembre 1995 (1995-09-01), pages 1569-1573, XP000533273 Tokyo (jp)

## Description

La présente invention concerne un procédé de préparation d'une composition pharmaceutique sous forme de microsphères à libération prolongée d'un principe actif hydrosoluble. L'invention a également pour objet des microsphères susceptibles d'être obtenues par la mise en oeuvre de ce procédé, présentant une libération continue de principe actif sur une période de plus de deux mois, avantageusement d'au moins trois mois.

De nombreuses compositions pharmaceutiques, sous forme de microsphères à base de polymères et copolymères biodégradables, contenant des composés pharmacologiquement actifs, et conçues pour une libération contrôlée et prolongée desdits composés, ont été décrites dans divers documents de l'état de la technique antérieure. De telles compositions pharmaceutiques présentent un grand intérêt pour le traitement de diverses maladies nécessitant une libération continue et prolongée de principe actif.

Néanmoins, les formulations développées jusqu'à présent pour la mise au point de ce type de compositions présentent divers inconvénients et peu d'entre elles parviennent jusqu'au stade des essais cliniques.

Un des problèmes majeurs liés à ces formulations à libération prolongée est la libération d'une quantité importante de principe actif pendant les premières heures qui suivent l'administration de la composition pharmaceutique. Il est communément fait référence à une telle libération sous le terme d'effet "burst" ou de libération "burst". Cette libération conduit généralement à une brusque augmentation des concentrations plasmatiques du médicament, ce qui débouche dans de nombreux cas sur des problèmes toxicologiques inacceptables pour l'être humain. Cette libération "burst" conduit également à une réduction de la durée d'activité de la composition pharmaceutique, du fait de la libération brusque et rapide d'une quantité importante dudit principe actif suite à l'administration de la composition.

Un second problème réside dans le fait que l'on obtient généralement des taux d'encapsulation peu efficaces en utilisant les procédés de microencapsulation habituels, en particulier lorsque le principe actif est un médicament soluble dans l'eau.

Un troisième problème qu'il est nécessaire de résoudre lors de la mise au point de ces formulations est l'instabilité des principes actifs face aux conditions rigoureuses employées lors de la fabrication des microsphères, telles que des températures élevées ou une mise en contact prolongée du principe actif avec des solvants organiques durant l'étape d'évaporation du solvant.

Plusieurs essais ont été mis en oeuvre afin de résoudre ces divers problèmes. Ainsi, des additifs tels que les sucres, les huiles, la cire, les protéines, les polymères, les sels, ou les acides, ont été utilisés dans la préparation de compositions pharmaceutiques sous forme de microsphères. Ces additifs, qui agissent comme des substances retenant le médicament dans la microsphère, permettent d'augmenter l'efficacité du procédé de microencapsulation et même éventuellement de protéger le principe actif durant le procédé, en jouant le rôle d'agents stabilisants.

Néanmoins, l'inclusion de ces additifs dans les microsphères peut conduire à des problèmes d'interaction entre les additifs et le principe actif ou la matrice à base de polymères, induisant ainsi des problèmes en matière de toxicologie et d'activité pharmacologique du médicament. En outre, les additifs, qui retiennent le principe actif à l'intérieur des microsphères pendant le procédé de fabrication, influent sur le profil de libération du principe actif contenu dans les microsphères, pouvant empêcher une libération continue dudit principe actif suite à l'administration des microsphères.

D'autres procédés de microencapsulation ont également été mis au point afin de tenter d'augmenter l'efficacité de la microencapsulation du principe actif au sein des microsphères, en se fondant sur l'utilisation de mélanges de solvants organiques, mais de tels procédés conduisent à des problèmes de stabilité du principe actif au cours du procédé de fabrication des microsphères.

D2 décrit la préparation de microsphères biodégradables contenant l'ovalbumine en tant que protéine modèle. Différents types de polymères polylactide (PLA) et poly-lactide-co-glycolide (PLGA) peuvent être utilisés. Cependant, les microsphères obtenues dans D2 présentent un effet « burst » élevé au bout de 3-4 jours après leur administration.

Par conséquent, il existait un besoin de mettre au point un procédé de préparation d'une composition pharmaceutique sous forme de microsphères à base de polymères et copolymères biodégradables, conçues pour une libération prolongée d'un principe actif hydrosoluble, ne présentant pas les inconvénients des compositions décrites dans les documents de l'état de la technique antérieure ou des compositions développées jusqu'à présent.

La présente invention vient combler ce besoin. La Demanderesse a ainsi découvert de manière surprenante que l'élaboration de microsphères, à base de principe actif et de copolymère matriciel biodégradable du type d,l-lactide-co-glycolide ayant un poids moléculaire spécifique et un ratio acide lactique/acide glycolique spécifique, par un procédé rapide du type émulsion multiple-évaporation de solvant, sans utiliser un quelconque additif ou un agent de modulation comme cela est le cas dans le brevet FR 2 718 642, permettait d'obtenir des microsphères présentant une libération continue et soutenue de principe actif sur une période de plus de deux mois, tout en présentant un effet "burst" restreint. Un tel procédé d'encapsulation, qui emploie des conditions douces et peu agressives pour le médicament, permet en outre de préserver la stabilité dudit médicament et d'obtenir une répartition homogène du médicament au sein de la microsphère obtenue.

Le principe physique d'émulsion multiple pour encapsuler des principes actifs hydrosolubles a notamment été décrit dans le brevet US 3 523 906. De manière générale, dans un procédé de ce type par émulsion multiple E/H/E et évaporation du solvant, le principe actif hydrosoluble est tout d'abord solubilisé dans la phase interne d'une première émulsion E/H, puis dans un second temps, cette première émulsion est à son tour émulsionnée dans une phase aqueuse externe.

La demanderesse a découvert de manière surprenante que l'utilisation d'un agent osmotique lors de l'étape d'émulsification de la première émulsion dans la phase aqueuse externe permettait d'obtenir une efficacité d'encapsulation particulièrement élevée par augmentation de la teneur de principe actif encapsulé au sein de la matrice polymérique, et d'influer sur la taille des microsphères.

L'objet de la présente invention, relatif à un procédé d'encapsulation rapide, très efficace et peu agressif pour le principe actif, permet ainsi d'obtenir des microsphères, à base de principe actif hydrosoluble et de copolymère matriciel du type d,l-lactide-co-glycolide, présentant une libération continue et soutenue de principe actif sur une période de plus de deux mois, de préférence d'au moins trois mois, tout en présentant un effet "burst" faible dans les heures suivant l'administration de la composition.

La présente invention a ainsi pour objet un procédé de préparation d'une composition pharmaceutique sous forme de microsphères à libération prolongée d'un principe actif hydrosoluble, caractérisé en ce qu'il comprend la succession d'étapes suivantes :
- dissolution du principe actif dans une quantité appropriée d'eau, ladite dissolution du principe actif étant réalisée sans l'addition d'aucune substance qui retienne le principe actif, ni d'agent stabilisateur de l'émulsion et sans aucune opération destinée à augmenter la viscosité,
- émulsification de la solution aqueuse de principe actif ainsi obtenue avec une solution d'un copolymère matriciel d,l-lactide-co-glycolide, de poids moléculaire moyen compris entre 40 000 et 80 000 et ayant une proportion acide lactique/acide glycolique comprise entre 50/50 et 80/20, dissous dans un hydrocarbure chloré, conduisant à une première émulsion microfine et homogène, la taille de ladite émulsion étant avantageusement inférieure à 1 µm,
- émulsification de ladite première émulsion ainsi obtenue dans une phase aqueuse externe, contenant 0,1 à 0,5% en poids de polysorbate 80, 1 à 25% en poids de polyvinylpyrrolidone, et 0,1 à 10% en poids de mannitol ou de chlorure de sodium,
- extraction-évaporation du solvant pour obtenir des microsphères que l'on récupère après filtration, lavage et séchage.

D'autres caractéristiques et avantages apparaîtront à la lecture de la description détaillée faite ci-après, notamment en s'appuyant sur quelques exemples de mise en oeuvre particuliers.

Le principe actif hydrosoluble utilisable dans le cadre du procédé selon la présente invention est avantageusement choisi dans le groupe constitué par les peptides, les protéines, les vaccins, les antibiotiques, les antidépresseurs, les analgésiques, les anti-inflammatoires et les cytostatiques. De manière encore plus avantageuse selon la présente invention, le principe actif est le 5-OxoPro-His-Trp-Ser-Tyr-D-Leu-Leu-Arg-ProNHEt ou l'un de ses sels. Ce principe actif est un analogue de l'hormone GnRH avec activité agoniste.

Dans le cadre du procédé selon la présente invention, la première étape de dissolution du principe actif dans de l'eau pour former une phase aqueuse interne est réalisée sans l'addition d'aucune substance qui retienne le principe actif, ni d'agent stabilisateur de l'émulsion et sans aucune opération destinée à augmenter la viscosité. Avantageusement selon la présente invention, le principe actif est dissous dans la phase aqueuse interne sans aucun additif ou adjuvant quelconque.

Les concentrations utilisées dans la phase aqueuse interne dans le cadre de la présente invention sont fonction de la solubilité du principe actif dans l'eau, des caractéristiques dudit principe actif et de la durée de libération que l'on désire obtenir. Avantageusement selon la présente invention, le principe actif est présent à une concentration comprise entre 0,01 et 95% en poids, avantageusement entre 0,5 et 40% en poids, par rapport au poids total de la phase aqueuse interne.

La formation de la première émulsion peut être notamment réalisée à l'aide d'un appareil à ultrasons ou d'un homogénéiseur.

Le copolymère matriciel utilisable selon la présente invention pour préparer des microsphères par E/H/E doit pouvoir être solubilisé dans un solvant volatil approprié, tel que les alcanes halogénés. Avantageusement selon la présente invention, le solvant est un hydrocarbure chloré tel que le chlorure de méthylène, le chloroforme, le chloroéthane, le dichloroéthane, ou le trichloroéthane. De manière encore plus avantageuse selon la présente invention, l'hydrocarbure chloré est le chlorure de méthylène.

Le copolymère matriciel d,l-lactide-co-glycolide utilisable dans le procédé selon la présente invention présente les avantages cumulés d'être insoluble dans l'eau, d'être biodégradable (un tel copolymère est absorbé sans s'accumuler dans les organes vitaux et est finalement totalement éliminé), d'être biocompatible avec l'organisme et d'être parfaitement toléré par lui, et finalement d'avoir une réponse inflammatoire minime.

Dans le cadre du procédé selon la présente invention, la solution du copolymère matriciel d,l-lactide-co-glycolide est obtenue par dissolution du copolymère dans un hydrocarbure chloré tel que le chlorure de méthylène, sans addition d'agent modulateur de libération. En effet, il a été découvert que l'utilisation d'un agent modulateur de libération n'était pas appropriée pour la fabrication de microsphères conçues pour une libération sur une période de temps de plus de deux mois.

La sélection effectuée sur le copolymère, avec un poids moléculaire spécifique et un ratio acide lactique/acide glycolique spécifique, combinée avec le fait de ne pas utiliser d'agent modulateur de libération dans le procédé objet de la présente invention présente ainsi l'avantage d'obtenir des microsphères présentant une libération continue et soutenue de principe actif sur une période de plus de deux mois, de préférence d'au moins trois mois, tout en présentant un effet "burst" restreint dans les heures suivant l'administration de la composition.

Les concentrations du polymère dissous dans la solution organique de chlorure de méthylène sont fonction du principe actif et de la vitesse de libération souhaitée. Avantageusement selon le procédé objet de la présente invention, le copolymère matriciel est présent à une concentration comprise entre 5 et 50% en poids, par rapport au poids total de la solution constituée du copolymère dissous dans l'hydrocarbure chloré.

Selon le procédé objet de la présente invention, la phase aqueuse externe contient un surfactant qui est le polysorbate 80, un agent augmentant la viscosité qui est le polyvinylpyrrolidone et un agent osmotique qui est le mannitol ou le chlorure de sodium. La phase aqueuse externe contient ainsi une solution de polysorbate 80, de polyvinylpyrrolidone et de mannitol ou de chlorure de sodium. Avantageusement selon la présente invention, la phase aqueuse externe contient une solution de polysorbate 80, de polyvinylpyrrolidone et de chlorure de sodium.

Le surfactant est présent à une concentration comprise entre 0,1 et 0,5% en poids, l'agent augmentant la viscosité est présent à une concentration comprise entre 1 et 25% en poids et l'agent osmotique est présent à une concentration comprise entre 0,1 et 10% en poids, par rapport au poids total de la phase aqueuse externe. La composition de la phase aqueuse externe est déterminante pour la formation de la deuxième émulsion (émulsification de la première émulsion), et elle est par conséquent déterminante pour la fabrication des microsphères. Elle contribue ainsi à la stabilisation rapide des microsphères, elle influe sur l'efficacité d'encapsulation des principes actifs et constitue un facteur essentiel pour contrôler la taille des microsphères finales et leur morphologie.

Durant l'étape d'extraction-évaporation du solvant, le solvant est rapidement éliminé à température ambiante et sous pression atmosphérique, selon un système évaporateur continu constitué d'une pente de longueur adéquate sur laquelle s'écoule en couche mince la suspension de microsphères.

Un tel système d'évaporation continue du solvant organique, qui augmente et favorise le contact entre l'émulsion et l'air, permet d'obtenir une stabilisation rapide de la matrice polymérique, ce qui a pour effet d'augmenter la stabilisation du principe actif tout en piégeant un fort pourcentage dudit principe au sein des microsphères (efficacité de l'encapsulation), et de conduire à une évaporation rapide du solvant, ce qui a pour effet de réduire le temps total de mise en oeuvre du procédé. La stabilisation rapide de la matrice polymérique obtenue lors de cette étape d'extraction-évaporation du solvant organique permet également d'obtenir une répartition homogène de la substance active dans toute la microsphère (permettant ainsi d'obtenir une faible concentration de principe actif encapsulé près de la surface de la microsphère), contribuant ainsi à diminuer le phénomène de libération "burst", suite à l'administration des microsphères. En outre, le fait de mettre en oeuvre le procédé à température ambiante et sous pression atmosphérique permet d'éviter des problèmes tels que l'altération des produits thermolabiles ou la cassure des microsphères quand le vide est utilisé lors de l'étape d'extraction-évaporation.

De façon plus détaillée, le procédé de fabrication des microsphères selon la présente invention comprend les étapes suivantes :
Une certaine quantité de principe actif est dissoute dans un volume d'eau. Cette solution s'émulsionne, à l'aide d'un appareil à ultrasons par exemple, dans un volume de chlorure de méthylène contenant un copolymère polylactide-co-glycolide, de poids moléculaire moyen compris entre 40,000 et 80,000 daltons et ayant une proportion de l'acide lactique à l'acide glycolique comprise entre 50/50 et 80/20. La première émulsion qui en résulte doit être micro-fine et homogène, permettant ainsi de répandre le principe actif sur toute la matrice polymérique, en assurant la reproductibilité des différents lots, sans avoir besoin d'utiliser des tensioactifs ni d'autres agents adjuvants. Une fois la première émulsion formée, on l'émulsionne à son tour dans une phase externe, constituée d'une solution aqueuse de polysorbate 80 en tant qu'agent de surface, de polyvinylpyrrolidone en tant qu'agent augmentant la viscosité et de mannitol ou NaCl en tant qu'agent osmotique, sous agitation pendant une courte période. Après cette étape, la double émulsion est diluée avec de l'eau, puis on fait passer la suspension dans des conditions atmosphériques sur un système évaporateur continu constitué d'une pente de longueur adéquate sur laquelle s'écoule en couche mince la suspension de microsphères. Ce système permet de favoriser le contact entre l'émulsion et l'atmosphère, ce qui réduit la durée d'évaporation du solvant organique, et d'augmenter la stabilisation du principe actif. Les microsphères obtenues de cette façon sont ensuite récupérées par filtration, lavées avec de l'eau et séchées par lyophilisation.

Les microsphères obtenues selon le procédé objet de la présente invention sont des microsphères à forte teneur en principe actif, permettant la libération en continu *in vivo* du médicament sur une période de plus de deux mois, de préférence d'au moins 3 mois, avec un faible effet "burst", après une administration parentérale des microsphères.

La présente invention a également pour objet les microsphères susceptibles d'être obtenues par la mise en oeuvre du procédé selon la présente invention, présentant une libération continue de principe actif sur une période de plus de deux mois, avantageusement sur une période d'au moins trois mois.

Avantageusement selon la présente invention, le principe actif est présent à une concentration comprise entre 0,5 et 20 % en poids, avantageusement entre 5 et 15 % en poids, par rapport au poids total des microsphères.

Avantageusement selon la présente invention, la taille des microsphères est inférieure à 250 µm, de manière encore plus avantageuse inférieure à 90 µm. Cette taille est adéquate pour l'administration des microsphères.

Les microsphères selon la présente invention sont avantageusement administrées par voie parentérale, de manière encore plus avantageuse sous la forme d'injections intramusculaires, sous-cutanées, intra-artérielles ou à l'endroit où se trouve une tumeur. Pour une administration adéquate, les microsphères sont de préférence dispersées dans un milieu aqueux standard avec des agents dispersants et des agents isotonisants.

Les exemples suivants (études in vivo et in vitro) sont donnés à titre non limitatif et illustrent la présente invention.

### Exemple 1 (comparatif) :

Des lots de microsphères à base de 5-OxoPro-His-Trp-Ser-Tyr-D-Leu-Leu-Arg-ProNHEt acétate sont préparés conformément au procédé de fabrication décrit dans la publication Advanced Drug Delivery Reviews, 28 (1997), 43-70, lequel diffère du procédé objet de la présente invention. La fabrication des microsphères est réalisée par dissolution d'une quantité appropriée de principe actif dans de l'eau, puis par une mise en émulsion de la solution aqueuse de principe actif ainsi obtenue avec une solution d'un polylactide (100% de lactide), de poids moléculaire moyen de 15,000, dans du chlorure de méthylène. La mise en émulsion est réalisée avec un agitateur, sous agitation vigoureuse. Une fois la première émulsion E₁/H formée, on l'émulsionne à son tour, afin d'obtenir une émulsion E₁/H/E₂, avec une solution aqueuse d'alcool polyvinylique (0,25%) en utilisant un mélangeur à grande vitesse. La double émulsion qui en résulte est ensuite doucement agitée pendant plusieurs heures afin d'évaporer le solvant organique. Les microsphères sont ensuite lavées, récupérées par centrifugation et lyophilisées. La charge finale des microsphères en médicament est de 9 à 11 % en poids.

Les différences principales entre les microsphères obtenues selon le procédé décrit dans le document de l'état antérieur de la technique cité ci-dessus et celles obtenues selon le procédé objet de la présente invention résident dans :
- le choix du polymère. Dans l'exemple 1, le polymère utilisé est un polylactide (100 % de lactide), de poids moléculaire moyen de 15,000. Il s'agit d'un polymère standard, selon ce document, pour une libération prolongée de 5-OxoPro-His-Trp-Ser-Tyr-D-Leu-Leu-Arg-ProNHEt acétate sur une période de trois mois.
- la composition de la phase externe aqueuse, qui est constituée par un alcool polyvinylique (0,25 %) dans l'exemple 1.
- le système d'évaporation du solvant organique. Dans l'exemple 1, la double émulsion est agitée pendant plusieurs heures.

### Exemple 2 :

En suivant le procédé de fabrication objet de la présente invention, le 5-OxoPro-His-Trp-Ser-Tyr-D-Leu-Leu-Arg-ProNHEt acétate est dissous dans de l'eau, puis la solution aqueuse de principe actif est émulsionnée, à l'aide d'un appareil à ultrasons, dans une solution de chlorure de méthylène contenant 15 % d'un copolymère d,l-lactide-co-glycolide, ce copolymère ayant un poids moléculaire moyen de 63,000 daltons (viscosité inhérente d'environ 0,6 dl/g) et une proportion de l'acide lactique à l'acide glycolique de 75/25. Une fois la première émulsion formée, on l'émulsionne à son tour avec une solution aqueuse constituée de 0,25 % de polysorbate 80, de 7 % de polyvinylpyrrolidone et de 5 % de mannitol, sous agitation, en utilisant un agitateur à hélices à pales. Après cette étape, on fait passer la double émulsion, dans des conditions atmosphériques, sur un système évaporateur continu constitué d'une pente de longueur adéquate sur laquelle s'écoule en couche mince la suspension de microsphères. A l'aide d'un tel système évaporateur, le solvant organique est rapidement évaporé, conduisant à une rapide stabilisation de la matrice polymérique. Les microsphères sont finalement récupérées par filtration, lavées avec de l'eau puis séchées sous lyophilisation. La charge finale des microsphères en médicament est de 9 à 11 % en poids.

### Exemple 2 bis :

Des microsphères sont élaborées en suivant le procédé décrit à l'exemple 2, en utilisant 5% de chlorure de sodium dans la phase aqueuse externe en tant qu'agent osmotique, au lieu de 5 % de mannitol.

### Exemple 3 :

L'étude de la libération *in vitro* de 5-OxoPro-His-Trp-Ser-Tyr-D-Leu-Leu-Arg-ProNHEt acétate à partir des microsphères préparées selon les exemples 1 et 2 est réalisée dans un tampon phosphate. 25 mg de chaque formulation sous forme de microsphères sont mis en suspension dans un milieu de libération composé de 5 ml de tampon phosphate (pH = 7,4), puis agités (rotation) pendant 4 jours à 37°C. A divers instants, la quantité de peptide libéré a été mesurée par chromatographie en phase liquide à haute pression (HPLC).

Les résultats de cette étude sont présentés dans le tableau 1. Ils démontrent que la composition préparée conformément au procédé objet de la présente invention (exemple 2) présente une libération "burst" bien inférieure à la libération "burst" des compositions préparées conformément au procédé de l'état antérieur de la technique de l'exemple 1. Ainsi, 4 jours après le début de l'étude, les microsphères de l'exemple 2 n'ont libéré que 3,4 % de la quantité totale de peptide présente dans les microsphères, alors que les microsphères de l'exemple 1 en ont libéré jusqu'à 22,6 %.

**Tableau 1 :**

| | % de peptide libéré | |
|---|---|---|
| Temps | Exemple 1 | Exemple 2 |
| 1 jour | 13,4 % | 1,0 % |
| 2 jours | 16,7 % | 1,4 % |
| 4 jours | 22,6 % | 3,4 % |

### Exemple 4 :

L'étude de la libération *in vivo* de 5-OxoPro-His-Trp-Ser-Tyr-D-Leu-Leu-Arg-ProNHEt acétate à partir des microsphères préparées selon les exemples 1, 2 et 2 bis est réalisée sur des rats.

Pour cette étude, les trois types de microsphères (exemples 1, 2 et 2 bis) sont mis en suspension dans un excipient aqueux standard, avant de procéder à leur administration sous-cutanée au niveau d'une zone dorsale préalablement rasée chez des rats. La dose administrée à chaque animal est de 3,6 mg de 5-OxoPro-His-Trp-Ser-Tyr-D-Leu-Leu-Arg-ProNHEt acétate microencapsulé. Certains rats sont sacrifiés trois jours après l'administration, alors que d'autres sont sacrifiés sept jours après l'administration. Une fois les rats morts, le site d'injection est alors excisé, puis les microsphères restantes au niveau du site, avec le tissu conjonctif contigu, sont récupérées. Le 5-OxoPro-His-Trp-Ser-Tyr-D-Leu-Leu-Arg-ProNHEt acétate restant est alors extrait, puis quantifié par chromatographie en phase liquide à haute pression (HPLC).

Les résultats de cette étude sont présentés dans le tableau 2. On peut constater qu'au bout de 3 jours, les microsphères de l'exemple 1 libèrent déjà 35,3 % de la quantité totale de peptide présente dans les microsphères, alors que les microsphères de l'exemple 2 n'en libèrent que 20,6 %.

**Tableau 2 :**

| | % de peptide libéré | | |
|---|---|---|---|
| Temps | Exemple 1 | Exemple 2 | Exemple 2 bis |
| 3 jours | 35,3 % | 20,6 % | 25,9 % |
| 7 jours | 41,6 % | 29,5 % | ----- |

On peut ainsi conclure que les résultats de cette étude *in vivo* concordent avec les résultats obtenus *in vitro* (exemple 3) et démontrent que les formulations préparées conformément au procédé de la présente invention présentent une libération "burst" beaucoup moins importante que la libération "burst" des compositions préparées conformément au procédé de l'état antérieur de la technique de l'exemple 1.

### Exemple 5 :

Afin de compléter les résultats précédents (exemples 3 et 4), une étude approfondie de la libération *in vivo* de principe actif pendant les premières 24 heures suite à l'administration des microsphères a été réalisée.

Dans ce test, la teneur sérique de 5-OxoPro-His-Trp-Ser-Tyr-D-Leu-Leu-Arg-ProNHEt acétate est évaluée chez des rats par chromatographie en phase liquide - spectrométrie de masse - spectrométrie de masse (CL/SM/SM), après l'administration sous-cutanée des formulations sous forme de microsphères préparées selon les exemples 1 et 2. Ainsi, 3,6 mg de 5-OxoPro-His-Trp-Ser-Tyr-D-Leu-Leu-Arg-ProNHEt acétate encapsulé sont injectés chez des rats et, à divers moments après l'administration, des échantillons de sang sont prélevés pour l'estimation de la teneur de 5-OxoPro-His-Trp-Ser-Tyr-D-Leu-Leu-Arg-ProNHEt acétate dans le sérum.

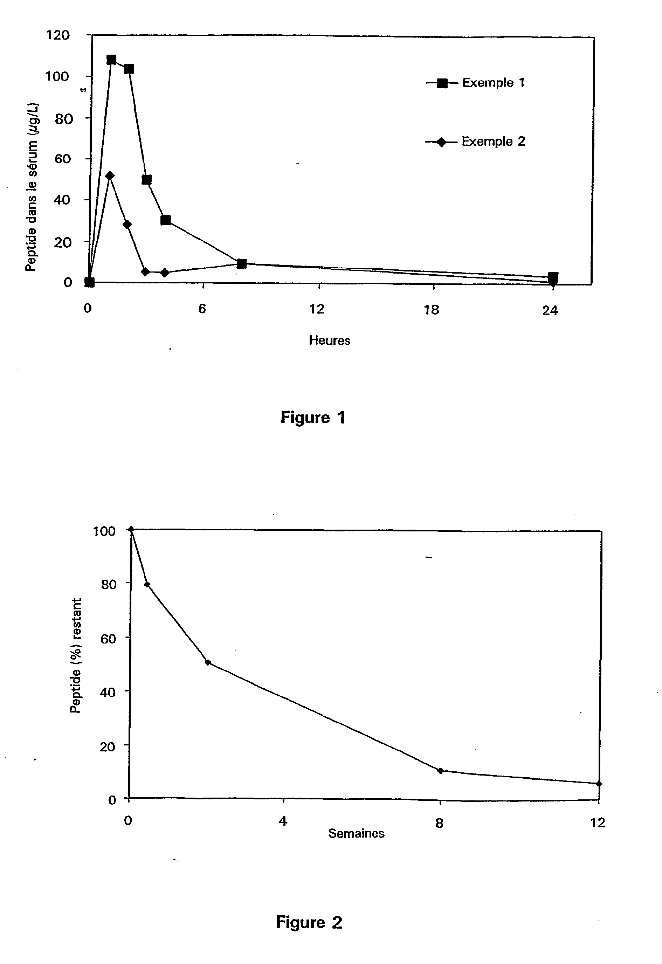

Les résultats de cette étude sont présentés sur la figure 1. Ainsi, une heure après l'administration des microsphères de l'exemple 1, la concentration de peptide dans le sérum est de 107,8 µg/l, alors que pour les microsphères de l'exemple 2, la concentration de peptide dans le sérum est de 51,8 µg/l. Après 2, 3, 4, 8 et 24 heures, les microsphères de l'exemple 1 présentent respectivement des concentrations sériques de 103,7 ; 50,1 ; 30,3 ; 9,5 et 3,8 µg/l, alors que les microsphères de l'exemple 2 présentent respectivement des valeurs de 28,0 ; 5,5 ; 4,5 ; 9,3 et 1,0 µg/l respectivement.

Ainsi, les résultats de cette étude sont en accord avec les résultats obtenus pour les exemples 3 et 4, puisque l'on constate que les microsphères de l'exemple 2 libèrent moins de peptide pendant les premières 24 heures (effet "burst") que les microsphères de l'exemple 1. Nous pouvons en conclure que lorsque les microsphères contiennent la matrice polymérique selon la présente invention, et qu'elles sont fabriquées en utilisant le procédé selon la présente invention, elles présentent une libération "burst" sensiblement inférieure aux microsphères préparées conformément au procédé de l'état antérieur de la technique après une administration *in vivo.*

### Exemple 6 :

Le profil complet de libération du 5-OxoPro-His-Trp-Ser-Tyr-D-Leu-Leu-Arg-ProNHEt acétate à partir des microsphères préparées selon l'exemple 2 est étudié chez des rats. L'objet de l'étude est de démontrer que, suite au phénomène de libération "burst" (qui a lieu systématiquement, mais qui est relativement faible par rapport à des microsphères décrites dans l'état antérieur de la technique), les microsphères obtenues selon le procédé de la présente invention libèrent le peptide en continu sur une période de plusieurs mois.

De manière similaire à l'exemple 4, les microsphères sont mises en suspension dans un véhicule aqueux standard pour leur administration sous-cutanée au niveau d'une zone dorsale préalablement rasée chez des rats. La dose administrée à chaque animal est de 3,6 mg de 5-OxoPro-His-Trp-Ser-Tyr-D-Leu-Leu-Arg-ProNHEt acétate encapsulé.

A divers moments sur une période de trois mois, des groupes de rats sont sacrifiés, le site d'injection est excisé, puis les microsphères restantes au niveau du site, avec le tissu conjonctif contigu, sont récupérées. Le 5-OxoPro-His-Trp-Ser-Tyr-D-Leu-Leu-Arg-ProNHEt acétate restant au niveau du site d'injection est alors extrait, puis quantifié par chromatographie en phase liquide à haute pression (HPLC).

Les résultats de cette étude sont présentés sur la figure 2. Ils montrent ainsi que les microsphères libèrent le peptide en continu sur une période d'au moins trois mois.

### Exemple 7 :

L'étude de l'activité biologique *in vivo* du 5-OxoPro-His-Trp-Ser-Tyr-D-Leu-Leu-Arg-ProNHEt acétate microencapsulé dans des microsphères préparées selon l'exemple 2 et l'exemple 2 bis est réalisée ici.

Le 5-OxoPro-His-Trp-Ser-Tyr-D-Leu-Leu-Arg-ProNHEt acétate est un analogue agoniste puissant de l'hormone LH-RH, qui stimule la sécrétion des gonadotrophines par l'hypophyse et la stéroïdogénèse dans les organes génitaux à des doses aiguës. Toutefois, lorsqu'il est administré de manière chronique, il produit, paradoxalement, des effets inhibiteurs antagonistes sur la gonadotrophine hypophysaire et la stéroïdogénèse testiculaire et ovarienne. Une administration initiale de 5-OxoPro-His-Trp-Ser-Tyr-D-Leu-Leu-Arg-ProNHEt acétate provoque une brusque augmentation des taux sériques de gonadotrophines et d'hormones sexuelles, mais dans le cas d'une exposition continue au médicament, ces taux d'hormones diminuent sensiblement jusqu'à parvenir en dessous des valeurs initiales de base après plusieurs jours d'administration, et ce phénomène demeure pendant la durée du traitement.

Par conséquent, afin de vérifier que la faible libération "burst" des formulations fabriquées selon les exemples 2 et 2 bis n'altère pas son activité pharmacologique, 3,6 mg de 5-OxoPro-His-Trp-Ser-Tyr-D-Leu-Leu-Arg-ProNHEt acétate micro-encapsulé dans ces microsphères sont injectés par administration sous-cutanée chez des rats et, à divers moments après l'administration, des échantillons de sang sont prélevés pour l'estimation de la testostérone sérique par dosage radio-immunologique.

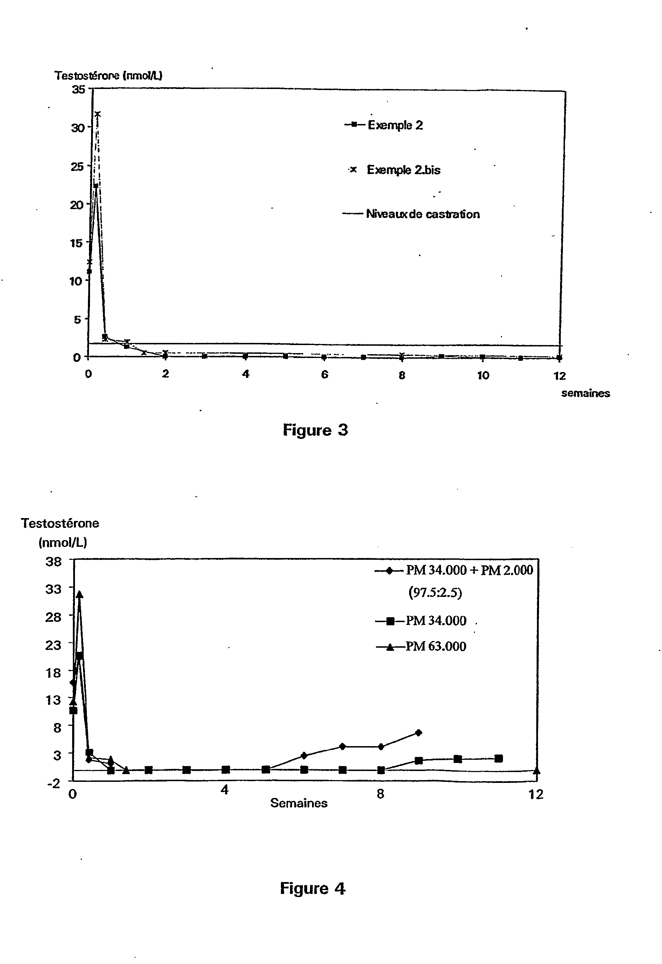

Les résultats de cette étude sont présentés sur la figure 3. Les résultats montrent ainsi un profil de testostérone sérique approprié : une brusque augmentation pendant les premiers jours, suivie d'une suppression de la concentration plasmatique de testostérone, et ils démontrent ainsi une activité biologique correcte du peptide libéré à partir de la formulation selon la présente invention.

L'objet des exemples 8 à 10 est de montrer les divers avantages que présentent les microsphères préparées conformément au procédé objet de la présente invention par rapport aux microsphères préparées selon le procédé décrit dans le brevet FR 2 718 642. Les différences principales existant entre la présente invention et l'invention décrite dans le brevet FR 2 718 642 sont les suivantes :
- Pas d'agent modulateur de libération utilisé dans le procédé de la présente invention.
- Ajout de mannitol ou de NaCl en tant qu'agent osmotique dans la phase aqueuse externe dans laquelle la première émulsion est émulsifiée.
- Sélection d'une plage de poids moléculaire (40 000 à 80 000) et d'une proportion acide lactique / acide glycolique (50/50 à 80/20) du copolymère d,l-lactide-co-glycolide (PLGA) dans la présente invention.

### Exemple 8 : Effet de l'agent de modulation de libération sur la libération in vitro

Des microsphères sont préparées en suivant le procédé de fabrication décrit dans l'exemple 2, en utilisant en tant que phase organique une solution de chlorure de méthylène contenant 30 % d'un copolymère d,l-lactide-co-glycolide, ayant un poids moléculaire moyen de 34,000 daltons (viscosité inhérente d'environ 0,4 dl/g) et une proportion de l'acide lactique à l'acide glycolique de 50/50 ainsi que différentes quantités (exemples 8.1, 8.2 et 8.3) d'un poly(d,l-lactide) ayant un poids moléculaire moyen de 2,000 daltons (PLA 2,000).
Exemple 8.1 : 0 % de PLA 2,000.
Exemple 8.2 : 2,5 % de PLA 2,000.
Exemple 8.3 : 5 % de PLA 2,000.
La phase aqueuse externe est une solution aqueuse composée de 0,25 % de polysorbate 80, de 4 % de polyvinylpyrrolidone et de 5 % de mannitol.

La libération initiale *in vitro* de 5-OxoPro-His-Trp-Ser-Tyr-D-Leu-Leu-Arg-ProNHEt acétate à partir des microsphères préparées selon les exemples 8.1, 8.2 et 8.3 est réalisée dans un tampon phosphate.

25 mg de chaque formulation sont mis en suspension dans un milieu de libération composé de 5 ml de tampon phosphate (pH = 7,4), puis agités (rotation) pendant 7 jours à 37°C. A divers instants, la quantité de peptide libéré est mesurée par chromatographie en phase liquide à haute pression (HPLC). Les résultats sont présentés dans le tableau 3 suivant.

**Tableau 3 :**

| Temps | Peptide libéré en % | | |
|---|---|---|---|
| Jours | Exemple 8.1 | Exemple 8.2 | Exemple 8.3 |
| 2 jours | 1,1 % | 1,1 % | 1,6 % |
| 4 jours | 1,7 % | 2,7 % | 2,9 % |
| 7 jours | 3,7 % | 11,8 % | 18,3 % |

Les résultats montrent, pour ces 3 cas de figure 8.1, 8.2 et 8.3, une libération "burst" très faible et montrent que l'agent de modulation (PLA) de libération a un effet d'accélération de la libération du principe actif, et ceci même à de faibles concentrations (2,5 %) de PLA, indiquant que l'utilisation de cet agent peut ne pas être adéquat pour la fabrication de microsphères conçues pour une libération prolongée (sur une période de trois mois).

### Exemple 9: Effet de l'agent de modulation de libération sur le profil pharmacodynamique et sélection du polymère pour une libération prolongée sur trois mois

L'effet de la composition polymérique des microsphères sur la libération du principe actif est étudié dans une étude pharmacodynamique réalisée chez des rats. Les microsphères sont préparées en suivant le procédé de fabrication décrit dans l'exemple 2, en utilisant différents types de polymère :
**Exemple 9.1 :** Mélange de 97,5 % d'un copolymère d,l-lactide-co-glycolide, ayant un poids moléculaire moyen de 34,000 daltons et une proportion de l'acide lactique à l'acide glycolique de 50/50, et de 2,5 % d'un poly(d,l-lactide) ayant un poids moléculaire moyen de 2,000 daltons (agent de modulation de libération).
**Exemple 9.2 :** Copolymère d,l-lactide-co-glycolide, ayant un poids moléculaire moyen de 34,000 daltons et une proportion de l'acide lactique à l'acide glycolique de 50/50.
**Exemple 9.3** : Copolymère d,l-lactide-co-glycolide, ayant un poids moléculaire moyen de 63,000 daltons et une proportion de l'acide lactique à l'acide glycolique de 75/25.

L'étude de la libération *in vivo* du 5-OxoPro-His-Trp-Ser-Tyr-D-Leu-Leu-Arg-ProNHEt acétate à partir des microsphères est suivie à travers son effet pharmacologique de suppression des taux plasmatiques de testostérone.

Les résultats de cette étude sont présentés sur la figure 4. Les résultats confirment ainsi que la présence de l'agent de modulation de libération accélère la libération du principe actif et que l'agent de modulation de libération ne peut donc pas être utilisé dans des formulations à libération très prolongée (taux de testostérone supprimés pendant 5 semaines).

Les résultats montrent également qu'un copolymère d,l-lactide-co-glycolide, ayant un poids moléculaire moyen de 34,000 daltons et une proportion de l'acide lactique à l'acide glycolique de 50/50, peut maintenir la suppression des taux de testostérone pendant 8 semaines, alors qu'un copolymère d,l-lactide-co-glycolide, ayant un poids moléculaire moyen de 63,000 daltons et une proportion de l'acide lactique à l'acide glycolique de 75/25, peut maintenir la castration pendant au moins trois mois.

### Exemple 10 : Effet de l'addition d'un agent osmotique dans la phase aqueuse externe

### Formulation 10.1 :

Des microsphères sont préparées en suivant le procédé de fabrication décrit dans l'exemple 2, en utilisant en tant que phase aqueuse externe une solution composée de 0,25 % de Polysorbate 80 et de 4 % de polyvinylpyrrolidone.

### Formulation 10.2 :

Des microsphères sont préparées en suivant le procédé de fabrication décrit dans l'exemple 10.1, en ajoutant entre 2,5 % et 5 % de mannitol dans la phase aqueuse externe en tant qu'agent osmotique.

### Formulation 10.3 :

Des microsphères sont préparées en suivant le procédé de fabrication décrit dans l'exemple 10.1, en ajoutant entre 2,5 % et 5 % de chlorure de sodium dans la phase aqueuse externe en tant qu'agent osmotique.

Les résultats de l'étude montrant l'influence de l'agent osmotique dans la phase aqueuse externe sont présentés dans le tableau 4 suivant :

**Tableau 4:**

| Exemple | Agent osmotique | Taille *µ*m | Teneur % |
|---|---|---|---|
| 10.1 | ----- | 59,3 | 7,7 |
| 10.2 | Mannitol 2,5 % | 50,4 | 10,5 |
| 10.2 | Mannitol 5 % | 43,8 | 10,7 |
| 10.3 | NaCl 2,5 % | 39,7 | 10,7 |
| 10.3 | NaCl 5 % | 32,7 | 10,4 |

Ainsi, la taille et la teneur en peptide des microsphères dépendent de la présence de l'agent osmotique dans la phase aqueuse externe. L'addition de mannitol ou de NaCl en tant qu'agent osmotique augmente l'efficacité d'encapsulation, c'est à dire le pourcentage de principe actif microencapsulé (teneur). Le type et la quantité de l'agent osmotique ajouté peuvent également permettre le contrôle de la taille des particules. Dans le cadre de la présente invention, le NaCl est avantageusement utilisé en tant qu'agent osmotique, car il a été mis en évidence que dans des conditions d'agitation et de viscosité identiques, le chlorure de sodium permet une plus grande réduction de la taille des particules que le mannitol, sans produire pour autant une diminution de l'efficacité d'encapsulation.

### Exemple 11 : Effet de la concentration de l'agent augmentant la viscosité (agent viscosifiant) présent dans la phase aqueuse externe sur la taille des particules

### Formulation 11.1 :

Des microsphères sont préparées en suivant le procédé de fabrication décrit dans l'exemple 2, mais sans principe actif, en utilisant en tant que phase aqueuse externe une solution composée de 0,25 % de Polysorbate 80, 5 % de mannitol et 6,8 % de polyvinylpyrrolidone.

### Formulation 11.2 :

Des microsphères sont préparées en suivant le procédé de fabrication décrit dans l'exemple 2, mais sans principe actif, en utilisant en tant que phase aqueuse externe une solution composée de 0,25 % de Polysorbate 80, 5 % de mannitol et 8,0 % de polyvinylpyrrolidone.

Les résultats de l'étude montrant l'influence de l'agent viscosifiant dans la phase aqueuse externe sont présentés dans le tableau 5 suivant :

**Tableau 5 :**

| Exemple | Agent viscosifiant | Taille µm |
|---|---|---|
| 11.1 | Polyvinylpyrrolidone 6,8% | 31,3 |
| 11.2 | Polyvinylpyrrolidone 8,0% | 25,4 |

L'exemple 10.2 élaboré avec une phase aqueuse externe composée de 0,25% de Polysorbate 80, 5% de mannitol et 4% de polyvinylpyrrolidone permettait d'obtenir une taille de particules de 43,8 µm. En augmentant la concentration d'agent viscosifiant, on peut constater qu'une diminution notable de la taille des particules est obtenue comme le montre le tableau 5 ci-dessus. Ainsi, la taille des microsphères dépend de la présence de l'agent viscosifiant dans la phase aqueuse externe, et de sa concentration.

## Revendications

1. Procédé de préparation d'une composition pharmaceutique sous forme de microsphères à libération prolongée d'un principe actif hydrosoluble, **caractérisé en ce qu'**il comprend la succession d'étapes suivantes :
- dissolution du principe actif dans une quantité appropriée d'eau, ladite dissolution du principe actif étant réalisée sans l'addition d'aucune substance qui retienne le principe actif, ni d'agent stabilisateur de l'émulsion et sans aucune opération destinée à augmenter la viscosité,
- émulsification de la solution aqueuse de principe actif ainsi obtenue avec une solution d'un copolymère matriciel d,l-lactide-co-glycolide, de poids moléculaire moyen compris entre 40 000 et 80 000 et ayant une proportion acide lactique/acide glycolique comprise entre 50/50 et 80/20, dessous dans un hydrocarbure chloré, conduisant à une première émulsion microfine et homogène,
- émulsification de ladite première émulsion ainsi obtenue dans une phase aqueuse externe contenant 0,1 à 0,5% en poids de polysorbate 80, 1 à 25% en poids de polyvinylpyrrolidone, et 0,1 à 10% en poids de mannitol ou de chlorure de sodium, extraction-evaporation du solvant pour obtenir des microsphères que l'on récupère après filtration, lavage et séchage.

2. Procédé selon la revendication 1, **caractérisé en ce que** le principe actif est choisi dans le groupe constitué par les peptides, les protéines, les vaccins, les antibiotiques, les antidépresseurs, les analgésiques, les anti-inflammatoires et les cytostatiques.

3. Procédé selon la revendication 2, **caractérisé en ce que** le principe actif est le 5-OxoPro-His-Trp-Ser-Tyr-D-Leu-Leu-Arg-ProNHEt ou l'un de ses sels.

4. Procédé selon l'une quelconque des revendications précédentes, **caractérisé en ce que** le principe actif est présent à une concentration comprise entre 0,01 et 95% en poids, avantageusement entre 0,5 et 40% en poids, par rapport au poids total de la phase aqueuse interne.

5. procédé selon l'une quelconque des revendications précédentes, **caractérisé en ce que** l'hydrocarbure chloré est le chlorure de méthylène.

6. Procédé selon l'une quelconque des revendications précédentes, **caractérisé en ce que** le copolymère matriciel est présent à une concentration comprise entre 5 et 50% en poids, par rapport au poids total de la solution constituée du copolymère dissous dans l'hydrocarbure chloré.

7. Procédé selon l'une quelconque des revendications précédentes, **caractérisé en ce que**, durant l'étape d'extraction-évaporation du solvant le solvant est rapidement éliminé à température ambiante et sous pression atmosphérique, selon un système évaporateur continu constitué d'une pente de longueur adéquate sur laquelle s'écoule en couche mince la suspension de microsphères.

8. Microsphères susceptibles d'être obtenues par la mise en oeuvre du procédé selon l'une quelconque des revendications 1 à 7, présentant une libération continue de principe actif sur une période de plus de deux mois, avantageusement sur une période d'au moins trois moins.

9. Microspheres selon la revendication 8, **caractérisées en ce que** le principe actif est présent à une concentration comprise entre 0,5 et 20 % en poids, avantageusement entre 5 et 15 % en poids, par rapport au poids total des microsphères.

10. Microsphères selon l'une des revendications 8 et 9, **caractérisées en ce que** leur taille est inférieure à 250 µm avantageusement inférieure à 90 µm.

11. Microsphères selon l'une des revendications 8 à 10, **caractérisées en ce qu'**elles sont administrées par voie parentérale, avantageusement sous la forme d'injections intramusculaires, sous-cutanées, intra-artérielles ou à l'endroit où se trouve une tumeur.

## Patentansprüche

1. Verfahren zur Herstellung eines Arzneimittels in Form von Mikrokügelchen zur verzögerter Freisetzung eines wasserlöslichen Wirkstoffes, **dadurch gekennzeichnet, dass** es die folgenden aufeinanderfolgenden Schritte umfasst:
- Lösen des Wirkstoffes in einer geeigneten Menge Wasser, wobei das Lösen des Wirkstoffes ohne Zugabe einer Substanz, die den Wirkstoff zurückhält, ohne einen Emulsionsstabilisator und ohne einen Schritt zur Erhöhung der Viskosität durchgeführt wird,
- Emulgieren der so erhaltenen wässrigen Wirkstofflösung mit einer Lösung einer D,L-Lactid-co-Glycolid-Copolymermatrix mit einem mittleren Molekulargewicht zwischen 40000 und 80000 und mit einem Milchsäure/Glykolsäure-Verhältnis zwischen 50/50 und 80/20, gelöst in einem chlorierten Kohlenwasserstoff, was zu einer ersten mikrofeinen und homogenen Emulsion führt,
- Emulgieren der so erhaltenen ersten Emulsion in einer externen wässrigen Phase, die 0,1 bis 0,5 Gew.-% Polysorbat 80, 1 bis 25 Gew.-% Polyvinylpyrrolidon und 0,1 bis 10 Gew.-% Mannitol oder Natriumchlorid enthält,
- Extraktion-Eindampfen des Lösungsmittels, um Mikrokügelchen zu erhalten, die nach Filtration, Waschen und Trocknen gewonnen werden.

2. Verfahren nach Anspruch 1, **dadurch gekennzeichnet, dass** der Wirkstoff ausgewählt ist aus der Gruppe bestehend aus Peptiden, Proteinen, Impfstoffen, Antibiotika, Antidepressiva, Analgetika, entzündungshemmenden Mitteln und Cytostatika.

3. Verfahren nach Anspruch 2, **dadurch gekennzeichnet, dass** der Wirkstoff 5-OxoPro-His-Trp-Ser-Tyr-D-Leu-Leu-Arg-ProNHEt oder ein Salz davon ist.

4. Verfahren nach einem der vorhergehenden Ansprüche, **dadurch gekennzeichnet, dass** der Wirkstoff in einer Konzentration zwischen 0,01 und 95 Gew.-%, vorzugsweise zwischen 0,5 und 40 Gew.-%, bezogen auf das Gesamtgewicht der internen wässrigen Phase, vorliegt.

5. Verfahren nach einem der vorhergehenden Ansprüche, **dadurch gekennzeichnet, dass** der chlorierte Kohlenwasserstoff Methylenchlorid ist.

6. Verfahren nach einem der vorhergehenden Ansprüche, **dadurch gekennzeichnet, dass** die Copolymermatrix in einer Konzentration zwischen 5 und 50 Gew.-%, bezogen auf das Gesamtgewicht der Lösung aus dem in dem chlorierten Kohlenwasserstoff gelösten Copolymer, vorliegt.

7. Verfahren nach einem der vorhergehenden Ansprüche, **dadurch gekennzeichnet, dass** während des Extraktions-Eindampfungsschrittes des Lösungsmittels das Lösungsmittel schnell bei Umgebungstemperatur und unter Atmosphärendruck mit Hilfe eines kontinuierlichen Eindampfsystems bestehend aus einem Gefälle mit angemessener Länge, auf dem die Mikrokügelchen-Suspension in dünner Schicht abfließt, entfernt wird.

8. Mikrokügelchen, erhältlich durch Durchführen des Verfahrens nach einem der Ansprüche 1 bis 7, welche eine kontinuierliche Wirkstoff-Freisetzung über einen Zeitraum von mehr als zwei Monaten, vorzugsweise über einen Zeitraum von mindestens drei Monaten, aufweisen.

9. Mikrokügelchen nach Anspruch 8, **dadurch gekennzeichnet, dass** der Wirkstoff in einer Konzentration zwischen 0,5 und 20 Gew.-%, vorzugsweise zwischen 5 und 15 Gew.-%, bezogen auf das Gesamtgewicht der Mikrokügelchen, vorliegt.

10. Mikrokügelchen nach einem der Ansprüche 8 und 9, **dadurch gekennzeichnet, dass** ihre Größe kleiner als 250 µm, vorzugsweise kleiner als 90 µm, ist.

11. Mikrokügelchen nach einem der Ansprüche 8 bis 10, **dadurch gekennzeichnet, dass** sie auf parenteralem Weg, vorzugsweise in Form von intramuskulären, subkutanen, intraarteriellen Injektionen oder Injektionen an der Stelle, an der sich ein Tumor befindet, verabreicht werden.

## Claims

1. A method for preparing a pharmaceutical composition in the form of microspheres with prolonged release of a water-soluble active principle, **characterized in that** it comprises the succession of steps below:
- dissolving the active principle in an appropriate amount of water, said dissolving of the active principle being carried out without the addition of any substance which retains the active principle, nor of agent for stabilizing the emulsion, and without any operation intended to increase the viscosity,
- emulsifying the aqueous solution of active principle thus obtained with a solution of a d,l-lactide-co-glycolide matrix copolymer of average molecular weight of between 40 000 and 80 000 and having a lactic acid/glycolic acid proportion of between 50/50 and 80/20, dissolved in a chlorinated hydrocarbon, resulting in a first microfine and homogeneous emulsion,
- emulsifying said first emulsion thus obtained, in an external aqueous phase containing 0.1 to 0.5% by weight of polysorbate 80, 1 to 25% by weight of polyvinylpyrrolidone and 0.1 to 10% by weight of mannitol or of sodium chloride,
- extracting-evaporating the solvent so as to obtain microspheres, which are recovered after filtration, washing and drying.

2. The method according to claim 1, **characterized in that** the active principle is chosen from the group consisting of peptides, proteins, vaccines, antibiotics, antidepressants, analgesics, antiinflammatories and cytostatics.

3. The method according to claim 2, **characterized in that** the active principle is 5-OxoPro-His-Trp-Ser-Tyr-D-Leu-Leu-Arg-ProNHEt or one of its salts.

4. The method according to any one of the preceding claims, **characterized in that** the active principle is present at a concentration of between 0.01 and 95% by weight, advantageously between 0.5 and 40% by weight, relative to the total weight of the internal aqueous phase.

5. The method according to any one of the preceding claims, **characterized in that** the chlorinated hydrocarbon is methylene chloride.

6. The method according to any one of the preceding claims, **characterized in that** the matrix copolymer is present at a concentration of between 5 and 50% by weight relative to the total weight of the solution consisting of the copolymer dissolved in the chlorinated hydrocarbon.

7. The method according to any one of the preceding claims, **characterized in that**, during the step consisting of extraction-evaporation of the solvent, the solvent is rapidly eliminated at ambient temperature and under atmospheric pressure, according to a continuous evaporating system consisting of a slope of adequate length, over which the suspension of microspheres flows in a thin layer.

8. A microsphere which can be obtained by implementing the method according to any one of claims 1 to 7, exhibiting continuous release of active principle over a period of more than two months, advantageously over a period of at least three months.

9. The microsphere according to claim 8, **characterized in that** the active principle is present at a concentration of between 0.5 and 20% by weight, advantageously between 5 and 15% by weight, relative to the total weight of the microspheres.

10. The microsphere according to either of claims 8 and 9, **characterized in that** it is less than 250 µm, advantageously less than 90 µm, in size.

11. The microsphere according to one of claims 8 to 10, **characterized in that** it is administered parenterally, advantageously in the form of intramuscular, subcutaneous or intra-arterial injections, or injections at the site of a tumor.
